# EUROPÄISCHE PATENTSCHRIFT

(11) **EP 1 762 586 B1**
(45) Veröffentlichungstag und Bekanntmachung des Hinweises auf die Patenterteilung: **07.03.2012**
(21) Anmeldenummer: 06018832.3
(22) Anmeldetag: 08.09.2006
(51) Int. Cl.: C08J 5/02, C08J 3/28, C08J 3/26, C08K 5/00, C08L 9/10, C08L 7/02, B29C 41/00, B29C 41/14, A47L 13/18, A61B 19/04, A61F 6/04, B01J 19/12

(54) **Verfahren zur Herstellung eines vernetzten Elastomers**
Method for producing a crosslinked elastomer
Procédé de production d'un élastomère réticulé

(30) Priorität: 12.09.2005 AT 14912005
(43) Veröffentlichungstag der Anmeldung: 14.03.2007
(73) Patentinhaber: Semperit Aktiengesellschaft Holding, 1031 Wien (AT)
(72) Erfinder: Schaller, Raimund, DI, Dr., 2620 Neunkirchen (AT); Holzner, Armin DI, Dr., 2630 Ternitz (AT); Ehrenfeldner, Richard, DI, 2630 Ternitz (AT); Höchtl, Michael, Dr., 1060 Wien (AT); Kern, Wolfgang, Dr., 8055 Seiersberg (AT); Stelzer, Franz, Dr., 8020 Graz (AT); Temel, Armin, Dl, 8010 Graz (AT)
(74) Vertreter: Chmilewsky-Lehner, Robert

(56) Entgegenhaltungen:
- EP-A- 0 232 016
- EP-A- 0 486 278
- EP-A- 1 193 551
- EP-A2- 0 792 925
- GB-A- 853 926
- US-A- 3 668 091
- US-A- 6 051 320
- US-A1- 2004 071 909
- US-B1- 6 329 444
- PHINYOCHEEP P., DUANGTHONG S.: "Ultraviolet-curable liquid natural rubber" JOURNAL OF APPLIED POLYMER SCIENCE, Bd. 78, 2000, Seiten 1478-1485, XP002449102
- TANGBORIBOONRAT P ET AL: "NEW EVIDENCE OF THE SURFACE MORPHOLOGY OF DEPROTEINIZED NATURAL RUBBER PARTICLES" POLYMER BULLETIN, SPRINGER, HEIDELBERG, DE, Bd. 41, Nr. 5, November 1998 (1998-11), Seiten 601-608, XP000787944 ISSN: 0170-0839

## Beschreibung

Die Erfindung betrifft die Verwendung einer Mischung umfassend zumindest einen Latex sowie gegebenenfalls weitere Hilfsstoffe zur Herstellung eines Handschuhs, Operationshandschuhs, Kondoms oder eines medizinischen Latexproduktes, ein Verfahren zur Herstellung eines medizinischen Latexproduktes aus einem vernetzten Elastomer, nach dem eine Mischung umfassend zumindest einen Latex und zumindest eine Starterkomponente zur Auslösung der Vernetzungsreaktion oder ein Latex der zumindest eine Starterkomponente bzw. Startergruppe trägt hergestellt wird, insbesondere in flüssiger Phase, sowie einen Handschuh, insbesondere medizinischer Handschuh oder Operationshandschuh, hergestellt aus zumindest einem, mit einer Starterkomponente vernetzten, durch Elastomermoleküle gebildeten, Elastomer.

Um Elastomeren die spezifischen elastischen Eigenschaften zu verleihen, müssen die Polymerketten des Elastomers zumindest partiell miteinander versetzt werden. Üblicherweise erfolgt die Vernetzung über Doppelbindungen, die sich entweder in der Hauptkette, wie beispielsweise bei Polyisoprene, Polybutadien, Styrol-Butadien-Kautschuk, Chloropren, Nitril-Butadien-Kautschuk, oder einer Seitenkette, wie z.B. bei EPDM, des Elastomers zu befinden. Großtechnisch wird die Vernetzung von Elastomeren, darunter auch Kautschuk-Latex, bislang auf drei verschiedene Arten durchgeführt.

Am verbreitetsten ist die Vernetzung, d.h. Vulkanisation, von Elastomeren mittels molekularen Schwefels, die auf Goodyear zurückgeht. Da die Vulkanisation mit Schwefel alleine zu lange Reaktionszeiten bedingt, werden Vulkanisationsbeschleuniger zugesetzt. Dabei handelt es sich um Organoschwefelverbindungen, wie Dithiocarbamate, Mercaptobenzothiazole oder Xanthogenate. Da die Vernetzung von Kautschuk-Latices bzw. Kautschuk-Copolymeren bei niedrigen Temperaturen durchgeführt werden muss, um den Latex nicht zu destabilisieren, ist der Einsatz von sehr reaktiven Vulkanisationsbeschleunigern essentiell.

Eine weitere Möglichkeit zur Vernetzung von Elastomeren besteht in der Reaktion mit organischen Peroxiden. Dabei werden Verbindungen, wie Dicumylperoxid oder Dibenzoylperoxid, thermisch in Radikale gespalten, welche die Vernetzung der Elastomerketten auslösen. Vorteilhaft dabei ist, wenn unter Luftausschluss gearbeitet wird, da Peroxide sauerstoffempfindlich sind, wozu häufig die Vernetzung in geschmolzenen Salzbädern erfolgt.

Neben den thermischen Vernetzungsverfahren existieren auch noch Strahlungsvernetzungsverfahren, in denen energiereiche Elektronenstrahlung oder Gammastrahlung eingesetzt wird. In diesen Verfahren werden häufig dem zu vernetzenden Kautschuk-Latex zusätzliche Reagenzien, wie z.B. Acrylate, zugesetzt. Ein derartiges Strahlungsvernetzungsverfahren ist z.B. aus der US 6,329,444 B bekannt. Dieses Dokument beschreibt insbesondere einen medizinischen Handschuh umfassend cis-1,4-Polyisopren, wobei dieser synthetische Kautschuk den Vorteil hat, dass er im Vergleich zum Naturkautschuk protein- und schwefelfrei ist. Hergestellt wird dieser medizinische Handschuh durch Tauchformen eines vorvernetzten cis-1,4-Polyisoprens. Die Vorvernetzung bzw. Vernetzung erfolgt, wie bereits erwähnt, mittels Elektronenstrahlung oder Gammastrahlung ohne chemische Sensibilisatoren. Es werden dabei Dosen von 20 Megarad bis 40 Megarad eingesetzt. Ebenso beschrieben wird in dieser US-B1 die peroxidische Vernetzung.

Aus der EP 1 193 551 A2 ist eine Beschichtung für Fotos oder Speichermedien bekannt, die aus einem UV-vernetzbaren Material, einem Latex sowie einem Fotoinitiator hergestellt wird.

Die EP 0 232 016 A2 beschreibt eine Beschichtung aus einem wässrigen Latex eines filmbildenden Copolymers, dem optional ein Fotoinitiator zugesetzt werden kann. Dieser wird unter Anderem zur Beschichtung von Holz, Papier, Kartonagen, Kunststoffen, Beton, Keramiken und Metallen verwendet.

Aus der EP 0 792 925 A2 ist eine Latexzusammensetzung mit verbesserter Trocknungsgeschwindigkeit bekannt, die durch Bestrahlung vernetzt wird. Die Zusammensetzung weist ein Polymer auf, dass zwischen 1 und 20 Gew.-% einer oder mehrerer Seitenketten aufweist, wobei diese Seitenketten das Produkt der Reaktion einer mono- oder polyfunktionellen Gruppe sind, die mit Säuren oder Hydroxylgruppen reagieren können. Diese Zusammensetzung kann optional einen UV-Fotoinitiator enthalten. Verwendet werden diese Latex-Zusammensetzungen für Beschichtungen, insbesondere für Holz und Holzprodukte, Metalle, Kunststoffe und Leder, sowie für Klebstoffe, Tinten oder Druckfarben und Dichtungsmaterialien.

Die EP 0 486 278 A1 beschreibt ebenfalls eine wasserbasierende, UV-härtbare Beschichtungszusammensetzung mit einer Latex-Polymeremulsion und einem multifunktionalen (Meth)Acrylat.

Aus der US 3,668,091 A ist ein Verfahren zum Bleichen von Carboxylsäureesther und/oder Epoxykomponenten durch Bestrahlung mit UV-Licht bekannt.

Aus Phinyocheep P., Duangthong S.: "Ultraviolet-Curable Liquid Natural Rubber" Journal of applied polymer science, Bd. 78, 2000, Seiten 1478-1485 ist es bekannt, mittels UV-Strahlung Naturgummi zu härten, wobei die Naturgummimoleküle ein fotosensitives Molekül aufweisen. Der flüssige Naturgummi wird oxidativ abgebaut zu einem Naturgummilatex mittels Phenylhydrazin/O2 Dieser wird in der Folge mit einem Gemisch aus Ameisensäure und Wasserstoffperoxid oxidiert. Als fotosensitives Molekül wird Acrylsäure zur Bildung von Acrylaten am Epoxyring verwendet. Die Bestrahlung erfolgt in Gegenwart eines Fotoinitiators.

Aus der GB 853 926 A ist es bekannt, einen Gummilatex durch hochenergetische Strahlung zu härten, wie beispielsweise durch Röntgenstrahlung, Gammastrahlung, Elektronenstrahlung, etc.

Aus Tangboriboonrat P., et al.: "New evidence of the surface morphology of deproteinized natural rubber particles" Polymer Bulletin, Springer, Heidelberg, DE, Band 41 Nr. 5, November 1998, Seiten 601-608 ist die Vernetzung von entprotionierten Naturgummilatexpartikeln durch Gammastrahlung bekannt.

Die US 6,329,444 B1 beschreibt medizinische Gegenstände aus Cis-1,4-Polyisoprenlatex, hergestellt mittels Tauchverfahren, wobei diese Gegenstände - unter anderem Handschuhe - im Vergleich zum Naturgummi ein geringeres Allergiepotential aufweisen. Die Vernetzung wird über Elektronenstrahlen oder Gammastrahlen bzw. strahlenlos mittels Peroxiden durchgeführt.

Die US 2004/0071909 A1 beschreibt dünnwandige Gummiartikel, hergestellt aus einem wässrigen Latex aus Naturgummi oder synthetischen Cis-1,4-Polyisopren durch peroxidische Vernetzung im Salzbad in Gegenwart von Schwefel enthaltenden Vulkanisiermittel. Durch die Vermeidung von aminhältigen Zusatzstoffen werden nitrosaminfreie Gummiartikel hergestellt.

Die US 6,051,320 A beschreibt dünnwandige Naturgummiprodukte, beispielsweise Handschuhe, hergestellt aus einer Latexemulsion, die neben dem Naturgummi schwefelfreie, sauerstoffabgebende Vernetzungsmittel und festigkeitssteigernde Zusätze enthält. Die Vernetzung erfolgt also peroxidisch.

Aufgabe vorliegender Erfindung ist es, ein strahlenchemisches Vernetzungsverfahren für einen Latex und ein damit hergestelltes Latexprodukt bereit zu stellen, welches im Vergleich zu herkömmlichen, bekannten strahlenchemischen Vernetzungsverfahren einfacher durchführbar ist.

Diese Aufgabe der Erfindung wird - jeweils eigenständig - mit der eingangs genannten Verwendung dadurch gelöst, dass die Mischung weiters zumindest einen Fotoinitiator enthält, der durch Bestrahlung mit elektromagnetischer Strahlung im ultravioletten (UV-Licht) und/oder sichtbaren Spektralbereich eine reaktive Spezies bildet, zur Auslösung der Vernetzungsreaktion, wobei der zumindest eine Fotoinitiator bzw. zumindest eine Fotoinitiatorgruppe gegebenenfalls Teil der Latexmoleküle ist, bei dem erfindungsgemäßen Verfahren dadurch, dass eine Mischung entsprechend einem der Ansprüche 1 bis 21 eingesetzt wird und die Latexmischung mit UV-Licht und/oder sichtbaren Licht bestrahlt wird, und durch einen Handschuh hergestellt nach dem erfindungsgemäßen Verfahren, bei dem die Starterkomponente bzw. die bei der Fotolyse entstehenden Zersetzungsprodukte aus der Starterkomponente an den Elastomermolekülen kovalent gebunden sind.

Von Vorteil ist dabei, dass durch die Vermeidung von Elektronen- oder Gammastrahlung, wie sie im Stand der Technik zur Vernetzung von Latices verwendet wird, das Vernetzungsverfahren an sich einfacher zu gestalten ist, insbesondere da keine besonderen Aufwendungen hinsichtlich Schutz vor ionisierender Strahlung, etc. notwendig sind. Die fotochemische Vernetzung ist weniger gefährlich und besser regelbar. In der Mischung werden keine konventionellen, schwefelhaltigen Beschleuniger, wie z.B. Dithiocarbamate oder Mercaptobenzothiazole, verwendet, wodurch das Sensibilisierungs- bzw. Hautreizungspotential bei Verwendung des hergestellten Latex für den Medizinbereich zumindest verringert ist. Es sind auch bei der Mischung bzw. den erfindungsgemäßen Handschuhen keine Verfärbungen beobachtbar, wenn diese mit Kupferflächen in Kontakt kommen, wie dies bei thermisch vernetzten, Dithiocarbamat aufweisenden Mischungen der Fall ist. Durch die kovalente Bindung der Fotoinitiatormoleküle bzw. der bei der Fotolyse entstehenden Zersetzungsprodukte aus der Fotoinitiatormoleküle an den Elastomermolekülen wird die Gefahr der Migration aus dem fertigen Produkt verringert, wodurch wiederum die Gefahr von Sensibilisierungen bzw. Hautreizungen infolge Verwendung dieser Artikel verringert ist. Darüber hinaus konnte festgestellt werden, dass die nach dem erfindungsgemäßen Verfahren hergestellten Latexprodukte eine sehr gute Alterungsbeständigkeit zeigen.

Es ist möglich einen Spektralbereich für die Vernetzung zu verwenden, mit Wellenlängen ausgewählt aus einem Bereich mit einer unteren Grenze von 150 nm und einer oberen Grenze von 600 nm. Damit ist dieses Verfahren sehr universell auf verschiedenste Latextypen anwendbar, ohne dass die Vorrichtung für die Durchführung des Verfahrens einer speziellen Anpassung bedarf.

Andererseits ist es möglich, zur Erhöhung der Spezifität der Reaktion, einen Spektralbereich zu verwenden, mit Wellenlängen, die ausgewählt sind aus einem Bereich mit einer unteren Grenze von 250 nm und einer oberen Grenze von 475 nm bzw. mit einer unteren Grenze von 275 nm und einer oberen Grenze von 400 nm. Es können damit gegebenenfalls auftretende Nebenreaktionen bedingt durch ein breiteres Energiespektrum und damit der Anregung anderer chemischer Funktionalitäten als der gewünschten, zumindest teilweise verhindert werden. Darüber hinaus ist es damit möglich, das Verfahren effizienter zu gestalten, indem hoch wirksame Fotoinitiatoren ausgewählt werden.

Prinzipiell ist für das erfindungsgemäße Vernetzungsverfahren jeder Fotoinitiator einsetzbar, der im ultravioletten und/oder sichtbaren Spektralbereich, insbesondere im an den UV-Bereich anschließenden Blaubereich des sichtbaren Spektralbereichs, eine entsprechende Reaktion zeigt. Für die Erhöhung des Umsatzes bei geeigneter Reaktionsführung werden jedoch Starterkomponenten, d.h. Fotoinitiatoren, bevorzugt, die ausgewählt sind aus einer Gruppe umfassend Verbindungen die unter Einfluss von UV-Strahlung in Radikale spalten, insbesondere Benzoinderivate, wie z.B. (2-Carboxyethyl) benzoin methyl ether, Benzoin ethyl ether ethoxy -1,2-diphenylethanon, Benzoin isopropyl ether propoxy -1,2- diphenylethanon, Benzoin isobuthyl ether isobutoxy -1,2- diphenylethanon, Mischungen aus Bezoin n-buthyl ether und Benzoin isobuthyl ether, Methylolbenzoinderivate, wie z.B. Vinyloxymethylbenzoin methyl ether, 4-Benzoyl-1,3-dioxolanderivate, Benzilketale, wie z.B. Benzildimethylketal, Dialkoxyacetophenone, wie z.B. α,α-Diethoxyacetophenon, α,α-Di-(n-butoxy)-acetophenon, Hydroxyalkylphenone, wie z.B. 1-Hydroxy-cyclohexyl-phenyl-keton, 2-Hydroxy-2-methyl-1-phenyl-propan-1-on, 1-Hydroxy-cyclohexyl-phenyl-keton mit 2-Hydroxy-2-methyl-1-phenyl-propan-1-on, 1-[4-(2-Hydroxyethoxy)phenyl]-2-hydroxy-2-methyl-propan-1-on, 1-Hydroxy-cyclohexyl-phenyl-keton mit Benzophenon, Aminoalkylphenone, wie z.B. 2-Methyl-1-[4-(methylthio)phenyl]-2-morpholino-propan-1-on, 2-Benzyl-2-dimethylamino-1-(4-morpholinophenyl)-butan-1-on, Acylphosphinoxide, wie z.B. 2,4,6-Trimethylbenzoyldiphenyl-phisphin oxid, 2,4,6-Trimethylbenzoyl-phenyl-phosphinsäureethylester, Bis(2,4,6-trimethylbenzoyl)-phenyl-phospinoxid, o-Acyl-α-Oximinoketone, Peroxide, wie z.B. Dibenzoylperoxid, Hydroperoxide, wie z.B. Cumolhydroperoxid, Perester, wie z.B. tert-Butyl-Perester von aromatischen Carbonsäuren, Alcyloxim-ester, Ketosulfide, wie z.B. Phenacylsulfide, Disulfide, wie z.B. Dibenzoyldisulfid, Dithiocarbamate, wie z.B. Diphenyldithiocarbamat, halogenierte Acetophenonderivate, Phenylglyoxylate, wie z.B. Methyl α-oxo benzenacetat, Halogenverbindungen, wie z.B. Alkylchloride, Alkylbromide, α-Halogenketone, wie z.B. Di- und Trichloroacetophenon, Hydrazone, cyclische Acetale, wie z.B. 1,3-Dioxalan, hierzu analoge Schwefelverbindungen, wie z.B. 1,3-Dithialan, Phenylester aliphatischer Carbonsäuren, sowie die entsprechenden Anilide, fotolabile organische Azide, wie z.B. Bis-azidobenzalcyclohexanon, Triphenylsulfoniumhexafluorophosphate, Diphenyliodonium Tetraflouroborate, Disulfone, wie z.B. Diphenyldislfon, Imimosulfonate, Thiosulfonate, wie z.B. S-Phenylbenzolthiosulfonat, zu genannten Schwefelverbindungen analoge Selenverbindungen, wie z.B. Diselenide, sowie Mischungen daraus, wie z.B. zumindest ein Benzoinderivat und/oder zumindest ein Methylolbenzoinderivat und/oder zumindest ein 4-Benzoyl-1,3-dioxolanderivat und/oder zumindest ein Benzilketal und/oder zumindest ein Dialkoxyacetophenon und/oder zumindest ein Hydroxyalkylphenon und/oder zumindest ein Aminoalkylphenon und/oder zumindest ein Acylphosphinoxid und/oder zumindest ein o-Acyl-α-Oximinoketon und/oder zumindest ein Peroxid und/oder zumindest ein halogeniertes Acetophenonderivat und/oder zumindest ein Phenylglyoxylat mit zumindest einem Methylolbenzoinderivat und/oder zumindest einem 4-Benzoyl-1,3-dioxolanderivat und/oder zumindest einem Benzilketal und/oder zumindest einem Dialkoxyacetophenon und/oder zumindest einem Hydroxyalkylphenon und/oder zumindest einem Aminoalkylphenon und/oder zumindest einem Acylphosphinoxid und/oder zumindest einem o-Acyl-α-Oximinoketon und/oder zumindest einem Peroxid und/oder zumindest einem halogenierten Acetophenonderivat und/oder zumindest einem Phenylglyoxylat, bzw. Verbindungen die unter Einfluss von UV-Strahlung Wasserstoff unter Bildung von Radikalen abstrahieren, insbesondere Benzophenonderivate, wie z.B. 2,4,6-Trimethyl-benzophenon mit Benzophenon (80%:20%), 4-Chlorobenzophenon, [4-[4-Methylphenylthio)phenyl]-phenylmethanon, 3,3'-Dimethyl-4-methoxy benzophenon, Methyl 2-benzoylbenzoat, 4-Phenyl-benzophenon, 4,4'-Bis(dimethylamino)-benzophenon, Michler's Ketone, Xanthonderivate, Thioxanthonderivate, wie z.B. Mischungen von 2-Chloro- mit 4-Chloro-thoxanthon, 2,4-Dimethylthioxanthon, 2,4-Diethoxythioxanthon, 1-Chloro-4-propoxy-thioxanthon, Anthrachinonderivate, Benzil, Diinon-Verbindungen, sowie Mischungen daraus, wie z.B. zumindest ein Benzophenonderivat und/oder zumindest ein Michler's Keton und/oder zumindest ein Xanthonderivat und/oder zumindest ein Thioxanthonderivat und/oder zumindest ein Anthrachinonderivat und/oder Benzil und/oder zumindest eine Diinon-Verbindung mit zumindest einem Benzophenonderivat und/oder zumindest einem Michler's Keton und/oder zumindest einem Xanthonderivat und/oder zumindest einem Thioxanthonderivat und/oder zumindest einem Anthrachinonderivat und/oder Benzil und/oder zumindest einer Diinon-Verbindung, oder aber auch oligomere Fotoinitiatoren, Fotoinitiatoren mit Vinyl- und/oder Acrylgruppen, Makromoleküle, insbesondere polymerisierte oder copolymerisierte Fotoinitiatoren, wie z.B. oligo [ 2-hydroxy-2-methyl-1-[4-(1-methylvinyl) phenyl] propanone], polymerisationsfähige Fotoinitiatoren, polymergebundene Fotoinitiatoren, Fotoinitiatoren, welche unter Bestrahlung an eine Polymer- bzw. Kautschukmatrix binden, sowie polymerisierte Vinyl-, Allyl-, Acryl- oder Methacrylderivate von: Benzoinderivaten, Methylolbenzoinderivaten, 4-Benzoyl-1,3-dioxolanderivaten, Benzilketalen, Dialkoxyacetophenonen, Hydroxyalkylphenonen, Aminoalkylphenonen, Acylphosphinoxiden, o-Acyl-α-Oximinoketonen, Peroxiden, halogenierten Acetophenonderivaten, Phenylglyoxylaten, Benzophenonderivaten, Michler's Ketonen, Xanthonderivaten, Thioxanthonderivaten, Anthrachinonderivaten, Benzil, Diinon-Verbindungen, sowie Mischungen daraus, wie z.B. zumindest ein oligomerer Fotoinitiator und/oder zumindest ein Fotoinitiator mit Vinyl- und/oder Acrylgruppen und/oder zumindest ein Makromolekül, insbesondere polymerisierte oder copolymerisierte Fotoinitiatoren, wie z.B. oligo [ 2-hydroxy-2-methyl-1-[ 4-(1-methylvinyl) phenyl] propanone] und/oder zumindest ein polymerisationsfähiger Fotoinitiator und/oder zumindest ein polymergebundener Fotoinitiator und/oder zumindest ein Fotoinitiator, der unter Bestrahlung an eine Polymer- bzw. Kautschukmatrix bindet und/oder zumindest ein polymerisiertes Vinyl-, Allyl-, Acryl- oder Methacrylderivat von: Benzoinderivaten, Methylolbenzoinderivaten, 4-Benzoyl-1,3-dioxolanderivaten, Benzilketalen, Dialkoxyacetophenonen, Hydroxyalkylphenonen, Aminoalkylphenonen, Acylphosphinoxiden, o-Acyl-α-Oximinoketonen, Peroxiden, halogenierten Acetophenonderivaten, Phenylglyoxylaten, Benzophenonderivaten, Michler's Ketonen, Xanthonderivaten, Thioxanthonderivaten, Anthrachinonderivaten, Benzil, Diinon-Verbindungen mit zumindest einem oligomeren Fotoinitiator und/oder zumindest einem Fotoinitiator mit Vinyl- und/oder Acrylgruppen und/oder zumindest einem Makromolekül, insbesondere polymerisierte oder copolymerisierte Fotoinitiatoren, wie z.B. oligo [ 2-hydroxy-2-methyl-1-[ 4-(1-methylvinyl) phenyl] propanone] und/oder zumindest einem polymerisationsfähigen Fotoinitiator und/oder zumindest einem polymergebundenen Fotoinitiator und/oder zumindest einem Fotoinitiator, der unter Bestrahlung an eine Polymer- bzw. Kautschukmatrix bindet und/oder zumindest einem polymerisierten Vinyl-, Allyl-, Acryl- oder Methacrylderivat von: Benzoinderivaten, Methylolbenzoinderivaten, 4-Benzoyl-1,3-dioxolanderivaten, Benzilketalen, Dialkoxyacetophenonen, Hydroxyalkylphenonen, Aminoalkylphenonen, Acylphosphinoxiden, o-Acyl-α-Oximinoketonen, Peroxiden, halogenierten Acetophenonderivaten, Phenylglyoxylaten, Benzophenonderivaten, Michler's Ketonen, Xanthonderivaten, Thioxanthonderivaten, Anthrachinonderivaten, Benzil, Diinon-Verbindungen. Die einzelnen Starterkomponenten können für sich einzeln eingesetzt werden oder in Mischungen, wobei auch Mischungen von zumindest einer Verbindungen nach Anspruch 5 und/oder zumindest einer Verbindungen nach Anspruch 6 und/oder zumindest einer Verbindungen nach Anspruch 7 mit zumindest einer Verbindungen nach Anspruch 5 und/oder zumindest einer Verbindungen nach Anspruch 6, gegebenenfalls mit zumindest einer Verbindung nach Anspruch 8, und/oder zumindest einer Verbindungen nach Anspruch 7, gegebenenfalls mit zumindest einer Verbindung nach Anspruch 8, möglich sind ohne dass die einzelnen Verbindungen bzw. Kombinationen von Verbindungen zur Vermeidung von Wiederholungen an dieser Stelle ausdrücklich genannt werden.

Daneben oder zusätzlich zu diesen Starterkomponenten können auch anorganische Verbindungen als Starterkomponenten bzw. Fotokatalysatoren verwendet werden, wie z.B. Metalloxide, beispielsweise ZnO, TiO₂, Metallsalze und Metallkomplexe, beispielsweise FeCl₃, ZnCl₂, TiCl₄, CoCl₂, MnCl₂, Metallcarbonyle, beispielsweise Manganpentacarbonyl, Benzol-Chrom-Tricarbonyl, Dichromate, Ferrocen in Verbindung mit Halogenverbindungen oder Pseudohalogenverbindungen, beispielsweise CCl₄ Phenylacylthiocyanat, Metallkomplexe, Ester von anorganischen Säuren, beispielsweise tert-Butylnitrit, Phenylcyanat, Phenylthiocyanat, Benzylthiocyanat, Phenylacylthiocyanat, Acetylacetonate von Co(II), Mn(III), Fe(III), Cu(II).

Nach einer weiteren Ausführungsvariante ist vorgesehen, die Starterkomponente auszuwählen aus einer Gruppe umfassend Fotoinitiatoren, welche mit sichtbarem Licht angeregt werden können, insbesondere Titanocene, 1,2-Diketone, wie z.B. Benzil, Derivate von Acylphosphinoxiden, substituierte Derivate von Stilben, Distyrylbenzol, sowie Mischungen daraus, wie z.B. zumindest ein Titanocen und/oder zumindest ein 1,2-Diketon, wie z.B. Benzil, und/oder zumindest ein Derivat von Acylphosphinoxid und/oder zumindest ein substituiertes Derivat von Stilben und/oder Distyrylbenzol mit zumindest einem Titanocen und/oder zumindest einem 1,2-Diketon, wie z.B. Benzil, und/oder zumindest einem Derivat von Acylphosphinoxid und/oder zumindest einem substituiertes Derivat von Stilben und/oder Distyrylbenzol.

Auch dabei sind wiederum Mischungen mit zumindest einer der oben bereits genannten Verbindungen möglich.

Darüber hinaus ist es möglich zur Erhöhung der Reaktivität die Starterkomponenten in Kombination mit zumindest einem Wasserstoffdonor, wie z.B. einem Amin oder Thiol, zu verwenden

Vorzugsweise wird als Starterkomponente 2,4,6-Trimethylbenzoylphenylphosphinsäureethylester und/oder 2-Hydroxy-2-methyl-1-phenylpropanon eingesetzt.

Für die Herstellung von medizinischen Latexprodukten, insbesondere Handschuhen, ist es von Vorteil, wenn für die Vernetzungsreaktion des Latex bzw. der Latexmischung im Hinblick auf ausgewogene mechanische Eigenschaften, d.h. insbesondere eine gewünschte Festigkeit bei entsprechender Relaxation des Handschuhs, d.h. Abbau des Druckes bzw. der Kraft auf die Hand nach der Dehnung infolge des Anziehens des Handschuhs und damit Vermeidung eines "Einschnürgefühls" beim Träger bzw. um die entsprechende Taktilität für den Handschuhträger zu gewährleisten, wenn die zumindest eine Starterkomponente oder ein Gemisch aus mehreren Starterkomponenten in einem Anteil zugesetzt wird, der ausgewählt wird aus einem Bereich mit einer unteren Grenze von 0,05 Gew.-% und einer oberen Grenze von 20 Gew.-%, bezogen auf den Feststoffgehalt des Latex an der Mischung.

Zur weiteren Verbesserung dieser Effekte ist gemäß Ausführungsvarianten vorgesehen, dass die zumindest eine Starterkomponente oder ein Gemisch aus mehreren Starterkomponenten in einem Anteil zugesetzt wird, der ausgewählt wird aus einem Bereich mit einer unteren Grenze von 0,1 Gew.-% und einer oberen Grenze von 10 Gew.-%, bezogen auf den Feststoffgehalt des Latex an der Mischung bzw. dass die zumindest eine Starterkomponente oder ein Gemisch aus mehreren Starterkomponenten in einem Anteil zugesetzt wird, der ausgewählt wird aus einem Bereich mit einer unteren Grenze von 0,5 Gew.-% und einer oberen Grenze von 4 Gew.-%, insbesondere 1 Gew.-% oder 2 Gew.-% oder 3 Gew.-%, bezogen auf den Feststoffgehalt des Latex an der Mischung.

Um die Vernetzung besser gestalten zu können ist es von Vorteil der Mischung zumindest ein Vernetzungshilfsmittel zuzusetzen. Insbesondere kann damit das Reaktionsverhalten der Starterkomponente, d.h. des Fotoinitiators, positiv beeinflusst werden.

Als Vernetzungshilfsmittel wird bevorzugt zumindest ein Thiol, und/oder ein Selenol, wie z.B. 1,6 Hexandiselenol, insbesondere jeweils mehrfach funktionelle Verbindungen bzw. Derivate davon, wie z.B. Bisthiole, Tristhiole, Bisselenole, Trisselenole, verwendet, wobei gemäß einer Ausführungsvariante vorgesehen ist dass das Thiol ausgewählt aus einer Gruppe umfassend Trimethylolpropan-tris-(3-mercaptopropionat), 1,6-Hexandithiol, sowie Mischungen daraus. Durch den Einsatz von Thiolen bzw. Selenolen, insbesondere multifunktionellen Thiolen, kann die Vemetzungsgeschwindigkeit erhöht werden, indem die UV-Reaktion bzw. die Reaktion im sichtbaren Spektralbereich zumindest teilweise nach dem so genannten Thiol-En Reaktionsmechanismus abläuft. Es wird damit nicht nur die Vemetzungsgeschwindigkeit beeinflusst, sondern können damit auch Produkte hergestellt werden, die höhere bzw. bessere mechanische Kennwerte, wie z.B. die Festigkeit, oder aber auch der Modul 500 %, aufweisen.

Sämtliche Kombinationen von den genannten Fotoinitiatoren mit zumindest einem der genannten Vernetzungshilfsmittel sind, obwohl zur Vermeidung von Wiederholungen diese nicht explizit erwähnt sind, möglich und vom Schutzumfang mit umfasst.

Dabei es ist es von Vorteil, wenn das Vernetzungshilfsmittel in einem Anteil zugesetzt wird, der ausgewählt wird aus einem Bereich mit einer unteren Grenze von 0,05 Gew.-% und einer oberen Grenze von 20 Gew.-%, bezogen auf den Feststoffgehalt des Latex an der Mischung, bzw. zur weiteren Verstärkung oben genannter Effekte, wenn das Vernetzungshilfsmittel in einem Anteil zugesetzt wird, der ausgewählt wird aus einem Bereich mit einer unteren Grenze von 0,1 Gew.-% und einer oberen Grenze von 10 Gew.-%, bezogen auf den Feststoffgehalt des Latex an der Mischung oder gemäß einer weiteren Ausführungsvariante nach der vorgesehen ist, dass das Vernetzungshilfsmittel in einem Anteil zugesetzt wird, der ausgewählt wird aus einem Bereich mit einer unteren Grenze von 0,5 Gew.-% und einer oberen Grenze von 4 Gew.-%, bezogen auf den Feststoffgehalt des Latex an der Mischung.

Es ist aber auch möglich, das Vernetzungshilfsmittel in einem Anteil zuzusetzen der ausgewählt ist aus einem Bereich mit einer unteren Grenze von 0,5 Gew.-% bzw. 1 Gew.-% und einer oberen Grenze von 3 Gew.-% bzw. 1,5 Gew.-%.

Der Mischung bzw. in dem Verfahren kann zumindest ein weiteres Hilfsmittel zugesetzt werden bzw. verwendet werden, ausgewählt aus einer Gruppe umfassend, insbesondere mehrfach funktionelle, Acrylate, wie z.B. Hexandioldiacrylat (HDDA), Trimethylolpropantriacrylat (TMPTA), Verbindungen mit Vinyl- oder Allylgruppen, wie z.B. Triallyl-Cyanurat, Triallyl-Isocyanurat, sowie Mischungen daraus, wie z.B. zumindest ein genanntes, insbesondere mehrfach funktionelles, Acrylat mit zumindest einer genannten Verbindung mit Vinyl- oder Allylgruppen, um damit das Vernetzungsverhalten zu verbessern, wobei wiederum mehrfach funktionelle Verbindungen bevorzugt werden.

Dieses weitere Vernetzungshilfsmittel wird bevorzugt in einem Anteil zugesetzt, der ausgewählt wird aus einem Bereich mit einer unteren Grenze von 0,05 Gew.-% und einer oberen Grenze von 20 Gew.-%, bezogen auf den Feststoffgehalt des Latex an der Mischung bzw. gemäß einer Ausführungsvariante hierzu, der ausgewählt wird aus einem Bereich mit einer unteren Grenze von 0,1 Gew.-% und einer oberen Grenze von 10 Gew.-%, bezogen auf den Feststoffgehalt des Latex an der Mischung oder nach einer weiteren Ausführungsvariante, der ausgewählt wird aus einem Bereich mit einer unteren Grenze von 0,5 Gew.-% und einer oberen Grenze von 4 Gew.-%, insbesondere 1 Gew.-% oder 2 Gew.-% oder 3 Gew.-%, bezogen auf den Feststoffgehalt des Latex an der Mischung.

Es ist auch möglich der Mischung zumindest einen Sensibilisator zuzusetzen, um die Lichtenergie besser auf den Fotoinitiator übertragen zu können und damit die Vernetzungsreaktion insgesamt zu beschleunigen bzw. den Ablauf positiv zu beeinflussen bzw. um damit auch die Möglichkeit zu schaffen, Fotoinitiatoren zu verwenden die in einem anderen Absorptionsbereich absorbieren als dieser für die gewünschte Reaktion vorteilhaft wäre.

Dabei ist es von Vorteil, wenn dieser zumindest eine Sensibilisator ausgewählt ist aus einer Gruppe umfassend organische Farbstoffe wie Eosin, anorganische Pigmente wie Zink-Phthalocyanin oder Titanoxide, sowie Mischungen daraus, da diese Verbindungen, insbesondere mit dem erfindungsgemäß eingesetzten Fotoinitiatoren, eine entsprechende Wechselwirkung zeigen.

Der Anteil des Sensibilisators liegt bevorzugt in einem Bereich mit einer unteren Grenze von 0,1 % und einer oberen Grenze von 50 % des Anteils der Starterkomponente(n), bzw. einem Bereich mit einer unteren Grenze von 10 % und einer oberen Grenze von 40 % des Anteils der Starterkomponente(n) oder einem Bereich mit einer unteren Grenze von 15 % und einer oberen Grenze von 25 % des Anteils der Starterkomponente(n), wodurch die Menge an zugesetzten Sensibilisator auf die Menge des verwendeten Fotoinitiator abgestimmt ist und somit die Gefahr, unverbrauchte Sensibilisatormoleküle im fertigen Latexprodukt zu haben, und damit gegebenenfalls eine Sensibilisierung bzw. Hautreizung hervorzurufen, zu verringern.

Vorzugsweise wird die zumindest eine Starterkomponente und/oder das zumindest eine Vernetzungshilfsmittel und/oder der zumindest eine Sensibilisator vor der Zugabe zu dem zumindest einen Latex zu einer Voremulsion oder Vordispersion voremulgiert oder vordispergiert und kann zu diesem Zweck, um das Dispergier- bzw. Emulgierverhalten dieser Komponenten zu verbessern, zumindest einen Emulgator bzw. zumindest ein Dispergierhilfsmittel der erfindungsgemäßen Mischung für das Vernetzungsverfahren zugesetzt werden, wobei als Emulgator bzw. Dispergierhilfsmittel besonders bevorzugt ein Tensid verwendet wird, insbesondere Polyethylenglykol-sorbitan-monolaurat. Es wird damit der Eintrag dieser Komponenten in die, insbesondere flüssige, Latexphase erleichtert bzw. kann damit auch eine "Gleichverteilung" dieser Komponenten in ihrer gesamten flüssigen Phase erreicht werden, wodurch höhere Reaktionsgeschwindigkeiten, d.h. höhere Umsätze pro Zeiteinheit, und damit eine Verkürzung des Verfahrens, erreicht werden kann.

Diese Voremulsion bzw. Vordispersion kann dem Latex bzw. Latexgemisch zumindest teilweise vor der Vernetzungsreaktion zugesetzt werden. Ebenso ist es möglich diese zumindest teilweise während der Vernetzungsreaktion zu zudosieren. Es kann damit auf das Vemetzungsverhalten, insbesondere den Start der Vernetzungsreaktion, unterschiedlicher Latextypen entsprechend reagiert werden bzw. kann der Verbrauch an Hilfskomponenten bzw. an Starterkomponenten an den jeweils gewünschten Vernetzungsgrad besser angepasst werden. Insbesondere ist damit auch der Vernetzungsgrad des Latex bzw. des Latexgemisches besser einstellbar.

Der zumindest eine erfindungsgemäß zu vernetzende Latex kann ausgewählt sein aus einer Gruppe umfassend Naturkautschuk (NR), Polyisoprene-Latex (IR), Nitrilkautschuk-Latex (NBR), Chloropren-Latex (CR), Styrol-Butadien Latex (SBR), Latices aus Ethylacrylat-Copolymeren (ACM), Latices aus Elastomeren welche durch Re-Emulgieren hergestellt werden, Latices aus funktionellen Copolymeren, wie z.B. fotoinitiatorhältige und oder carboxylierte, Latices hergestellt aus Polymer-Blends, sowie Mischungen daraus, wobei mit diesen Latices - obwohl die Verwendung des erfindungsgemäßen Verfahrens auf andere Laticestypen nicht ausgeschlossen sein soll -überraschender Weise entsprechend gute mechanische Eigenschaften bzw. entsprechende gute Eigenschaften erreicht werden konnten.

Dabei ist es von Vorteil, wenn der Latex mit einem Feststoffgehalt vernetzt wird, der ausgewählt ist aus einem Bereich mit einer unteren Grenze von 20 % und einer oberen Grenze von 60 %, insbesondere wenn der Latex mit einem Feststoffgehalt vernetzt wird, der ausgewählt ist aus einem Bereich mit einer unteren Grenze von 30 % und einer oberen Grenze von 50 %, vorzugsweise wenn der Latex mit einem Feststoffgehalt vernetzt wird, der ausgewählt ist aus einem Bereich mit einer unteren Grenze von 35 % und einer oberen Grenze von 45 %, da mit diesem Feststoffgehalt eine entsprechend gute Durchmischung der einzelnen Edukte und somit ein rascher Reaktionsablauf möglich ist.

Vorzugsweise wird die Vernetzungsreaktion in einem Fallfilmreaktor oder in einem Tauchreaktor durchgeführt, um bei vorbestimmbaren Schichtdicken des Reaktionsgemisches den Energieeintrag möglichst bis in die Kembereiche der Mischung, zu ermöglichen.

Als Energiequelle für elektromagnetische Strahlung wird bevorzugt eine Quecksilberlampe und/oder eine dotierte Quecksilberlampe und/oder eine Xenonlampe und/oder eine gepulste Xenonlampe und/oder eine Excimer-Lampe und/oder ein Laser, wie z.B. ein Excimer-Laser bzw. für die zumindest anteilsweise Vernetzungen im sichtbaren Blaubereich ein Laser, eine LED-Lichtquelle, verwendet, da mit diesen Lichtquellen bzw. Energiequellen eine entsprechend hohe Energieausbeute erzielbar ist.

Zur Erhöhung des Umsatzes bzw. um die Spezifität der Reaktion weiter zu erhöhen, kann bevorzugt monochromatische Strahlung verwendet werden und werden hierzu insbesondere Laser eingesetzt.

Wie bereits erwähnt, wird das Vernetzungsverfahren zur Herstellung eines medizinischen Handschuhs oder eines Operationshandschuhes angewandt. Insbesondere vorteilhaft kann hiermit auch ein nitrosaminfreier Handschuh, insbesondere aus Naturkautschuk, hergestellt werden.

Ebenso ist es möglich, dass an Elastomermolekülen zumindest das eine Vernetzungshilfsmittel, insbesondere ein multifunktionelles Thiol, kovalent gebunden, ist, um die Gefahr einer Hautreizung bei der Verwendung eines Handschuhs weiter zu minimieren.

Der Handschuh kann nitrosaminfrei und/oder beschleunigerfrei und/oder schwefelfrei hergestellt werden, wobei schwefelfrei in dem Sinne zu verstehen ist, dass kein freier Schwefel, wie er für die Schwefelvernetzung verwendet wird, vorhanden ist.

Der Reaktor kann mit einem Vorratsbehälter für die Mischung mit dem Latex strömungsverbunden sein, sodass also lediglich ein Anteil der zu vernetzenden Latexmischung durch den Reaktor strömt und damit, durch die Verringerung der Schichtdicke im Reaktor, die Umsetzungsgeschwindigkeit erhöht werden kann.

Schließlich kann dem Reaktor zumindest ein Tauchbecken nachgeordnet sein, d.h. dass dieser Teil einer Produktionsstraße für ein Tauchverfahren zur Herstellung von Handschuhen ist, wodurch die Wege zwischen den einzelnen Reaktoren verkürzt und damit die Produktionszeit insgesamt ebenfalls verkürzt werden kann.

Zum besseren Verständnis der Erfindung wird diese auch anhand der nachfolgenden Figuren näher erläutert.

Es zeigen jeweils in schematisch stark vereinfachter Darstellung:
- Fig. 1: eine erste Ausführungsvariante der Vorrichtung;
- Fig. 2: eine weitere Ausführungsvariante der Vorrichtung.

Es sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen.

Es sei an dieser Stelle angemerkt, dass erfindungsgemäß auch ein Latex vernetzt wird, der die funktionelle Gruppe, d.h. diejenige Gruppe die der Starterkomponente für die Fotolyse zugrunde liegt am Elastomermolekül trägt. Der Einfachheit halber wurden bzw. werden in der Beschreibung diese Gruppen nicht gesondert angeführt, da sich diese ohnehin für den Fachmann aufgrund der Lehre betreffend die Starterkomponenten von selbst erschliessen.

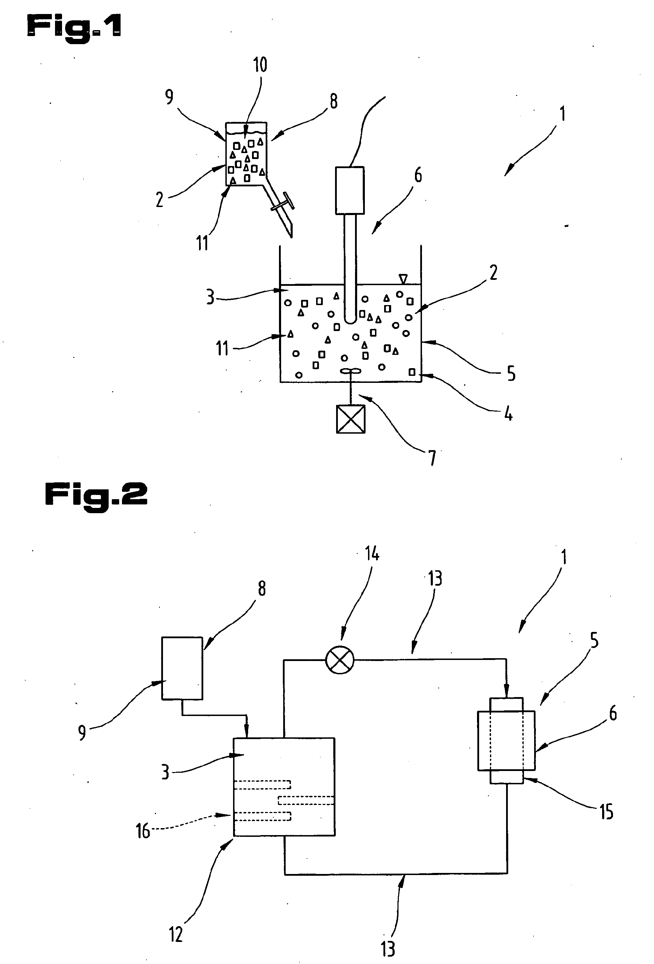

Fig. 1 zeigt eine Vorrichtung 1 für die Vernetzung eines Latex 2 bzw. eines Latexgemisches (in Fig. 1 mit Kreisen dargestellt). Der Latex 2 ist dabei Bestandteil einer Mischung 3, die neben dem Latex 2 zumindest eine Starterkomponente 4 (mit Quadraten dargestellt) in Form eines Fotoinitiators enthält. Die Starterkomponente 4 kann auch durch ein Gemisch 3 verschiedener Starterkomponenten 4 gebildet sein.

Diese Mischung 3 ist in einem Reaktor 5 die Vorrichtung 1 enthalten, wobei bei vorliegender Ausführung der Vorrichtung 1 der Reaktor 5 als Tauchreaktor ausgebildet ist.

In die Mischung 3 des Reaktors 5 taucht eine Energiequelle 6 in Form einer Lichtquelle ein. Diese Energiequelle 6 ist dazu ausgebildet, um elektromagnetische Strahlung im ultravioletten spektralen Bereich auszusenden. Gegebenenfalls kann diese Energiequelle 6 auch zur Aussendung von elektromagnetischen Wellen aus dem sichtbaren Bereich, insbesondere dem Blaubereich des sichtbaren Spektrums, ausgebildet sein. Auch Kombinationen sowohl des UV-Bereichs mit dem sichtbaren Bereich, insbesondere mit dem an den UV-Bereich anschließenden Blaubereich des Spektrums, sind möglich.

Mit Hilfe dieser Energiequelle 6 wird Energie in Form von Photonen auf die Starterkomponente 4, d.h. dessen Moleküle, übertragen und kann dadurch eine radikalische Spaltung der Moleküle der Starterkomponente 4 ausgelöst werden. Mit Hilfe der dabei entstehenden Radikale können in der Folge Doppelbindungen, welche in den Latexmolekülen vorhanden sind, beispielsweise in der Hauptkette und/oder in einer Seitenkette, entsprechend dem radikalischen Reaktionsmechanismus aufgebrochen werden und somit die Vernetzung über Startermoleküle oder direkt zwischen den Elastomermolekülen erfolgen. Die Energiequelle 6 ist dabei bevorzugt durch eine der bereits vorab genannten Energiequellen, also beispielsweise eine Quecksilberlampe oder durch einen Laser, gebildet.

Zur Homogenisierung der Mischung 3 im Reaktor 5 kann in diesem, wie in Fig. 1 angedeutet, ein Rührwerk 7 oder eine entsprechende Einrichtung mit vergleichbarer Funktion, angeordnet sein.

Des Weiteren besteht die Möglichkeit, benachbart zum Reaktor 5 ein Vorratsgefäß 8 anzuordnen, in dem die Starterkomponente 4 in einer Voremulsion bzw. Vordispersion gegebenenfalls mit einem entsprechenden Emulsionsmittel 10 nach einer Ausführungsvariante der Erfindung, vorgelegt wird. Als Emulsionsmittel 10 können dazu beispielsweise Tenside, wie bereits erwähnt, verwendet werden.

Daneben besteht nach einer weiteren Ausführungsvariante die Möglichkeit, der Mischung 3 und/oder Voremulsion 9 zumindest einen Hilfsstoff zuzusetzen. Dieser Hilfsstoff kann beispielsweise ein Vernetzungshilfsmittel 11 - in Fig. 1 dreieckförmig dargestellt - sein. Beispielsweise besteht die Möglichkeit als Vernetzungshilfsmittel ein Thiol und/oder ein Selenol vorzulegen. Bevorzugt weist das Thiol zwei oder mehrere SH-Gruppen auf, wodurch die Möglichkeit der so genannten Thiol-En Additionsreaktion, um damit Vernetzungsstellen im Polymer zu schaffen, zur Verfügung steht. Es kann damit eine Beschleunigung der Vernetzungsreaktion durchgeführt werden. Neben diesem Vernetzungshilfsmittel 11 können auch weitere Vernetzungshilfsmittel bzw. weitere Hilfsstoffe, wie z.B. Sensibilisatoren, Wasserstoffdonoren, diverse Prozessadditiva, wie z.B. Stabilisatoren, Antischaummittel, Dispergiermittel, Emulgatoren, Koagaliermittel, Vernetzungschemikalien, Farbstoffe und auch Füllstoffe in der Mischung 3 bzw. zumindest teilweise in der Voremulsion 9 vorhanden sein, wobei diese Reagenzien zumindest großteils aus dem Stand der Technik bekannt sind und sei der Fachmann an dieser Stelle an die einschlägige Literatur diesbezüglich verwiesen, beispielsweise die EP 0 856 294 A1 der Anmelderin bzw. auf die Veröffentlichung "Kautschuktechnologie" (Röthemeyer/Sommer, Carl Hanser Verlag 2001).

Die Voremulsion bzw. Vordispersion kann zumindest teilweise der Mischung 3 vor dem Start der Vernetzungsreaktion bzw. Vorvernetzungsreaktion für den Fall, dass eine mögliche Nachvernetzung von hergestellten Latexprodukten erfolgen soll, zugesetzt werden. Ebenso ist es möglich diese zumindest teilweise während der Reaktion dieser Mischung 3 beizufügen, beispielsweise in kleinen Mengen zuzugeben, beispielsweise zuzutropfen.

Die Energiequelle kann Licht im bereits beschriebenen Spektralbereich, also insbesondere zwischen 200 nm und 550 nm bzw. 250 nm und 475 nm bzw. 275 nm und 400 nm, aussenden.

Hinsichtlich der Starterkomponente bzw. möglicher Mischungen verschiedener Starterkomponenten sei an dieser Stelle auf obige Ausformungen verwiesen.

Sowohl die Energiequelle 6 als auch das Rührwerk 7 bzw. gegebenenfalls weitere Komponenten der Vorrichtung 1 können mit einer Steuer- und/oder Regeleinrichtung (nicht dargestellt), wie z.B. einem PC oder generell einer Datenverarbeitungsanlage, wirkungsverbunden sein, sodass gegebenenfalls eine Automatisierung bzw. ein Prozessablauf mit veränderlichen Reaktionsparametem vollautomatisch durchgeführt werden. Um dazu beispielsweise die Temperatur zu verändern, kann an oder in dem Reaktor 5 eine entsprechende Heiz- und/oder Kühleinrichtung aus dem Stand der Technik angeordnet werden.

Es ist des weiteren möglich, die Vernetzung bzw. die Vorvernetzung bei einem Druck, der unterschiedlich zum Atmosphärendruck ist, durchzuführen, beispielsweise bei Unterdruck, ebenso ist es möglich, die Vernetzungsreaktion bei Überdruck auszuführen und kann der Reaktor 5 diesbezüglich entsprechend ausgestaltet werden, also beispielsweise vakuumdicht, wobei sämtliche Einlässe bzw. Durchführungen durch die Wandungen des Reaktors 5 ebenfalls vakuumdicht ausgeführt sein sollten oder aber auch geeignet um Überdruckreaktionen durchzuführen, also beispielsweise mit verstärkten Wandungen.

Neben der Ausführungsvariante "Tauchreaktor", wie dies zu Fig. 1 dargestellt ist, besteht erfindungsgemäß u.a. auch die Möglichkeit den Reaktor 5 als Fallfilmreaktor auszubilden, wie dies in Fig. 2 dargestellt. Bei dieser Ausführungsvariante der Vorrichtung 1 umfasst diese neben dem Reaktor 5 auch fakultativ ein Vorratsgefäß 12, welches über eine Rohrleitung 13 strömungsverbunden mit dem Reaktor 5 ist, wobei die Rohrleitung 13 zusammen mit einer Umwälzeinrichtung 14, beispielsweise einer Pumpe, und dem Reaktor 5 sowie dem Vorratsgemäß 12, in dem die Mischung 3 vorgelegt ist, einen Kreislauf bildet, durch welchen die Mischung 3 aufgrund der Wirkung der Umwälzeinrichtung 14 befördert wird.

Der Fallfilmreaktor 5 weist an zumindest einer Oberfläche 15 die Energiequelle 6, also beispielsweise wiederum eine Quecksilberlampe oder einen Laser, auf, wobei in der Oberfläche 15 entsprechende Vorkehrungen, wie z.B. Sichtfenster, vorgesehen sein können, um das Eindringen der elektromagnetischen Strahlung in das Innere des Reaktors 5 zu ermöglichen. Des Weiteren ist es möglich diesen Reaktor 5 aus einem durchsichtigen Werkstoff, wie z.B. (Quartz)Glas oder Kunststoff, zumindest teilweise zu bilden.

Die Anordnung der Energiequelle kann aber auch im inneren des Fallfilmreaktors 5 erfolgen, sodass also die Mischung 3 von innen heraus belichtet wird

Auch bei dieser Ausführungsvariante besteht die Möglichkeit das Vorratsgefäß 8 wirkungsverbunden mit dem Vorratsgefäß 12 für die Mischung 3 anzuordnen und aus diesem Vorratsgefäß 8 die Voremulsion 9 bzw. Vordispersion im Vorratsgefäß 12 zuzuführen. Selbstverständlich besteht hier wiederum die Möglichkeit unmittelbar in diesem Vorratsgefäß 12 für die Mischung 3 eine Voremulsion bereitzustellen.

Wie in Fig. 2 strichliert dargestellt ist, beseht die Möglichkeit im Inneren des Vorratsgefäßes 12 Umlenkeinbauten 16, also beispielsweise Umlenkbleche, die gegebenenfalls Durchbrüche aufweisen können, vorzusehen, um eine Zwangsströmung im Vorratsgefäß 12 zu erzielen, um damit eine Durchmischung der Mischung 3 zu bewirken, sodass gegebenenfalls auf das Rührwerk 7 nach Fig. 1 verzichtet werden kann.

Generell ist auszuführen, dass der Reaktor 5 und/oder das Vorratsgefäß 8 bzw. das Vorratsgefäß 12 Teil einer Produktionsstraße einer Produktion von Latexprodukten sein kann, also beispielsweise von Handschuhen, insbesondere medizinischen Untersuchungshandschuhen oder Operationshandschuhen. Diese werden üblicherweise im Tauchverfahren hergestellt, wobei angemerkt sei, dass die Erfindung nicht nur auf Tauchartikel an sich beschränkt ist, sondern erfindungsgemäß Latexprodukte an sich durch Vernetzung des Latex bzw. der Latices im beanspruchten Spektralbereich durchgeführt werden kann.

Tauchverfahren für Handschuhe sind an sich aus dem Stand der Technik bekannt und sei der Fachmann beispielsweise auf die EP 0 856 294 A, insbesondere die Fig. 4 und 5 dieser EP-A sowie die zugehörigen Ausführungen in Spalte 14, Zeile 38 bis Spalte 18, Zeile 51 verwiesen, insbesondere in Hinblick auf die Ausführungen bezüglich der Koagulation, des Tauchens in Latex, diverser Waschvorgänge, diverser Nachbehandlungen, wie z.B. Chlorieren bzw. Halogenieren der Oberfläche der Handschuhe bzw. des Latex, die Herstellung von Oberflächenrauhigkeiten bzw. die Bereitstellung von puderfreien Handschuhen, etc.. Es soll damit auf unnötige Wiederholungen des Standes der Technik im Zusammenhang mit vorliegender Erfindung verzichtet werden und bildet daher die EP 0 856 294 A1 zumindest im besagten Umfang einen Teil der Offenbarung gegenständlicher Anmeldung.

Selbstverständlich ist es möglich, dass die Vorvernetzung bzw. Vernetzung des Latex bzw. der Latices gesondert, d.h. unabhängig von der weiteren Produktionsanlage für Latexprodukte, durchgeführt wird.

Neben Handschuhen können auch andere Latexprodukte erfindungsgemäß hergestellt werden, wie beispielsweise Kondome, diverse medizinische Latexprodukte, wie beispielsweise Katheter, Diaphragmen, Infusionsbeutel, medizinische Schläuche, Gewebekulturgefäße, etc.

Hinsichtlich der verwendeten Fotoinitiatoren, der diversen Hilfsstoffe sowie deren Konzentrationen bzw. Anteile an der Mischung 3 soll ebenso wie für die einsetzbaren Latices und deren Anteil an der Mischung 2 generell zur Vermeidung unnötiger Wiederholungen auf obige Ausführungen an dieser Stelle verwiesen sein.

Eingesetzte Feststoffe können im Rahmen der Erfindung in einem Solvens gelöst werden und/oder in Wasser emulgiert werden.

Es ist im Rahmen der Erfindung weiters möglich, nach der Formgebung eine Nachbehandlung durchzuführen, z.B. durch Erwärmen, Belichten oder Extrahieren.

Die Reißfestigkeiten der im Rahmen der Erfindung hergestellten Latexfilme können im Bereich von 25 N/mm² für Naturkautschuk liegen bei einer Reißdehnung von 800 % - 900 %.

Es ist weiters möglich die strahlenchemische Vernetzung sowohl kontinuierlich als auch diskontinuierlich durchzuführen.

Im Folgenden sind einige Beispiele angegeben, die im Rahmen der Erfindung durchgeführt worden sind.

### Beispiel 1:

1800 g High Ammonia Naturkautschuk (NR) Latex mit 40 % Feststoffgehalt werden mit 1 phr (parts per hundred parts of rubber, bezogen auf den Festkautschuk) Trimethylolpropan tris(3-mercaptopropionat) (als 50% Emulsion in Wasser mit 5 % Polyethylenglykol-sorbitan-monolaurat ("Tween 20") als Emulgator) und 1,5 phr Oligo [ 2-hydroxy-2-methyl-1-[4-(1-methylvinyl) phenyl] propanone] (Esacure KIP-150, Lamberti spa, als 25%ige Lösung in isoPropanol) versetzt und 2 Stunden mittels Magnetrührer gerührt. Anschließend werden 200 mL dieser Latexmischung in einem Vorratsgefäß vorgelegt und mittels einer Schlauchpumpe mit einer Förderleistung von etwa 250 mL/min im Kreislauf durch einen UV-Durchflussreaktor gepumpt. Der UV-Durchflussreaktor besteht aus einem zylindrischen Gefäß, welches aus Messing gefertigt und an der Innenseite mit einer Chromschicht versehen ist. Der Innendurchmesser beträgt 45 mm. In dieses Gefäß wird eine UV-Tauchlampe mit Kühler eingesetzt, wobei der Abstand zwischen Kühleroberfläche und Reaktorinnenwand 1 mm beträgt. Das Reaktionsgut (d.h. die Latexmischung) wird im unteren Teil des Reaktors zudosiert und im oberen Teil wieder abgezogen. Der UV-Durchflussreaktor ist mit einem UV-Strahler TQ-150 (150 Watt, ungefiltert, Hersteller Heraeus, Deutschland) mit Quarzglas-Kühler ausgestattet. Die Latexmischung wird 30 min im Kreislauf durch den UV-Durchflussreaktor gepumpt bis als Folge der UV-Bestrahlung der gewünschte Vernetzungsgrad erreicht ist. Die Reaktionstemperatur beträgt 20°C.

Der UV-vernetzte Latex wird aus dem Reaktor entnommen. Mithilfe einer Rakel werden aus diesem Latex Filme mit 125 Mikrometer Nassfilmdicke auf Zinkoxidpapier aufgestrichen und für eine Zeit von 120 Minuten bei 20°C an Luft getrocknet. Aus diesen Latexfilmen werden mit einem Stanzwerkzeug Scheiben (Durchmesser 25 mm) hergestellt und in Toluol bis zum Erreichen der Gleichgewichtsquellung eingelegt. Aus der Zunahme des Durchmessers der Scheibe kann der Quellungsgrad (in %) bestimmt werden. Er beträgt in diesem Fall ca. 80%.

Aus dem UV-vernetzten NR-Latex werden in einem industrieüblichen Koagulations-Tauchverfahren Filme hergestellt. Eine zylindrische Porzellanform wird zunächst in die Lösung eines Koagulationsmittels (10 Gewichtprozent CaCl₂ in Wasser) getaucht, nach 60 sec Trocknung bei 120°C in den UV-vernetzten Latex getaucht (30 sec) und anschließend für 20 Minuten bei 120°C getrocknet. Nach erfolgter Abkühlung auf Raumtemperatur wird der Latex-Film von der Porzellanform abgezogen. Die Filmdicke liegt typischerweise bei 250 µm. Diese Filme weisen im Zugversuch (nach ASTM Standard D412-98a) eine Reißfestigkeit zwischen 25 und 27 N/mm² sowie eine Reißdehnung zwischen 850 und 1000 % auf.

### Beispiel 2:

450 g High Ammonia Naturkautschuk (NR) Latex mit 40 % Feststoffgehalt werden mit 2 phr 2,4,6-Trimethylbenzoylphenylphosphinsäureethylester (Lucirin TPO-L, BASF, als 50% Emulsion in Wasser mit 5 % Polyethylenglykol-sorbitan-monolaurat ("Tween 20") als Emulgator) versetzt und 2 Stunden mittels Magnetrührer gerührt. Anschließend werden 200 mL dieser Latexmischung in einem Vorratsgefäß vorgelegt und mittels einer Schlauchpumpe mit einer Förderleistung von etwa 250 mL/min im Kreislauf durch den zuvor beschriebenen UV-Durchflussreaktor (siehe Beispiel 1) gepumpt.

Zur Bestimmung des Quellungsgrades werden Probekörper nach dem im Beispiel 1 dargestellten Verfahren hergestellt.

Nach der zuvor beschriebenen Methode der Gleichgewichtsquellung werden für den UVvemetzten NR-Latex die folgenden Vernetzungsgrade als Funktion der UV-Bestrahlungszeit bestimmt (siehe Tabelle 1):

**Tabelle 1:**

| | | | | | | |
|---|---|---|---|---|---|---|
| UV-Vernetzungszeit [min] | 5 | 10 | 15 | 20 | 25 | 30 |
| Quellungsgrad (Reifegrad) [in %] | 142 | 120 | 114 | 106 | 104 | 100 |

### Beispiel 3:

Synthetischer Polyisopren (IR) Latex (Kraton IR-401, erhalten von Fa. KRATON) wird mit 1 phr Nekal BX (erhalten von BASF, Deutschland) stabilisiert und auf 40 % Feststoffgehalt verdünnt. Der stabilisierte Polyisopren-Latex wird mit 0,5 phr Trimethylolpropan tris(3-mercaptopropionat) (als 50% Emulsion in Wasser mit 5 % Polyethylenglykol-sorbitan-monolaurat ("Tween 20") als Emulgator) und 0,5 phr 2-Hydroxy-2-methyl-1-phenylpropanon (Darocure 1173, Ciba Speciality Chemicals, als 50% Emulsion in Wasser mit 5 % Polyethylenglykol-sorbitan-monolaurat ("Tween 20") als Emulgator) versetzt und 2 Stunden mittels Magnetrührer gerührt. Anschließend werden 200 mL dieser Latexmischung in einem Vorratsgefäß vorgelegt und mittels einer Schlauchpumpe mit einer Förderleistung von etwa 250 mL/min im Kreislauf durch den zuvor beschriebenen UV-Durchflussreaktor (siehe Beispiel 1) gepumpt.

Zur Bestimmung des Quellungsgrades werden Probekörper nach dem im Beispiel 1 dargestellten Verfahren hergestellt.

Nach der zuvor beschriebenen Methode der Gleichgewichtsquellung werden für den UVvernetzten IR-Latex die folgenden Vernetzungsgrade als Funktion der UV-Bestrahlungszeit bestimmt (siehe Tabelle 2):

**Tabelle 2:**

| | | | | | | |
|---|---|---|---|---|---|---|
| Vernetzungs-Zeit [min] | 5 | 7 | 9 | 11 | 13 | 15 |
| Quellungsgrad (Reifegrad) [in %] | 120 | 108 | 102 | 96 | 92 | 92 |

Die Ausführungsbeispiele zeigen mögliche Ausführungsvarianten der Erfindung wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Ordnung halber sei abschließend darauf hingewiesen, dass zum besseren Verständnis des Aufbaus Vorrichtung 1 diese bzw. deren Bestandteile teilweise unmaßstäblich und/oder vergrößert und/oder verkleinert dargestellt wurden.

### Bezugszeichenaufstellung

- 1: Vorrichtung
- 2: Latex
- 3: Mischung
- 4: Starterkomponente
- 5: Reaktor

- 6: Energiequelle
- 7: Rührwerk
- 8: Vorratsgefäß
- 9: Voremulsion
- 10: Emulsionsmittel

- 11: Vernetzungshilfsmittel
- 12: Vorratsgefäß
- 13: Rohrleitung
- 14: Umwälzeinrichtung
- 15: Oberfläche

- 16: Umlenkeinbauten

## Patentansprüche

1. Verwendung einer Mischung umfassend zumindest einen Latex und zumindest einen Fotoinitiator, der durch Bestrahlung mit elektromagnetischer Strahlung im ultravioletten (UV-Licht) und/oder sichtbaren Spektralbereich eine reaktive Spezies bildet, zur Auslösung der Vernetzungsreaktion, wobei der zumindest eine Fotoinitiator bzw. zumindest eine Fotoinitiatorgruppe gegebenenfalls Teil der Latexmoleküle ist, sowie gegebenenfalls weitere Hilfsstoffe, zur Herstellung eines Handschuhs, Operationshandschuhs, Kondoms oder eines medizinischen Latexproduktes.

2. Verwendung nach Anspruch 1, **dadurch gekennzeichnet, dass** die Starterkomponente ausgewählt ist aus einer Gruppe umfassend Verbindungen die unter Einfluss von UV-Strahlung in Radikale spalten, insbesondere Benzoinderivate, Methylolbenzoinderivate, 4-Benzoyl-1,3-dioxolanderivate, Benzilketale, Dialkoxyacetophenone, Hydroxyalkylphenone, Aminoalkylphenone, Acylphosphinoxide, o-Acyl-α-Oximinoketone, Peroxide, halogenierte Acetophenonderivate, Phenylglyoxylate, sowie Mischungen daraus.

3. Verwendung nach einem der Ansprüche 1 oder 2, **dadurch gekennzeichnet, dass** die Starterkomponente ausgewählt wird aus einer Gruppe umfassend Verbindungen die unter Einfluss von UV-Strahlung Wasserstoff unter Bildung von Radikalen abspalten, insbesondere Benzophenonderivate, Michler's Ketone, Xanthonderivate, Thioxanthonderivate, Anthrachinonderivate, Benzil, Diinon-Verbindungen, sowie Mischungen daraus.

4. Verwendung nach einem der Ansprüche 1 bis 3, **dadurch gekennzeichnet, dass** die Starterkomponente ausgewählt ist aus einer Gruppe umfassend oligomere Fotoinitiatoren, Fotoinitiatoren mit Vinyl- und/oder Acrylgruppen, Makromoleküle, insbesondere polymerisierte oder copolymerisierte Fotoinitiatoren, wie z.B. oligo [2-hydroxy-2-methyl-1-[4-(1-methylvinyl) phenyl] propanone], polymerisationsfähige Fotoinitiatoren, polymergebundene Fotoinitiatoren, Fotoinitiatoren, welche unter Bestrahlung an eine Polymer- bzw. Kautschukmatrix binden, sowie polymerisierte Vinyl-, Allyl-, Acryl- oder Methacrylderivate von: Benzoinderivaten, Methylolbenzoinderivaten, 4-Benzoyl-1,3-dioxolanderivaten, Benzilketalen, Dialkoxyacetophenonen, Hydroxyalkylphenonen, Aminoalkylphenonen, Acylphosphinoxiden, o-Acyl-α-Oximinoketonen, Peroxiden, halogenierten Acetophenonderivaten, Phenylglyoxylaten, Benzophenonderivaten, Michler's Ketonen, Xanthonderivaten, Thioxanthonderivaten, Anthrachinonderivaten, Benzil, Diinon-Verbindungen, sowie Mischungen daraus.

5. Verwendung nach einem der Ansprüche 1 bis 4, **dadurch gekennzeichnet, dass** die Starterkomponente ausgewählt ist aus einer Gruppe umfassend Fotoinitiatoren, welche mit sichtbarem Licht angeregt werden können, insbesondere Titanocene, 1,2-Diketone, wie z.B. Benzil, Derivate von Acylphosphinoxiden, substituierte Derivate von Stilben, Distyrylbenzol, sowie Mischungen daraus.

6. Verwendung nach einem der Ansprüche 1 bis 5, **dadurch gekennzeichnet, dass** der Starterkomponente zumindest ein Wasserstoffdonor, wie z.B. einem Amin oder Thiol, zugesetzt ist.

7. Verwendung nach einem der Ansprüche 1 bis 6, **dadurch gekennzeichnet, dass** die Starterkomponente eine Verbindung ist, ausgewählt aus einer Gruppe umfassend 2,4,6-Trimethylbenzoylphenylphosphinsäureethylester, 2-Hydroxy-2-methyl-1-phenylpropanon.

8. Verwendung nach einem der Ansprüche 1 bis 7, **dadurch gekennzeichnet, dass** die zumindest eine Starterkomponente oder ein Gemisch aus mehreren Starterkomponenten in einem Anteil zugesetzt ist, der ausgewählt ist aus einem Bereich mit einer unteren Grenze von 0,05 Gew.-% und einer oberen Grenze von 20 Gew.-%, bezogen auf den Feststoffgehalt des Latex an der Mischung.

9. Verwendung nach einem der Ansprüche 1 bis 8, **dadurch gekennzeichnet, dass** der Mischung zusätzlich zumindest ein Vernetzungshilfsmittel zugesetzt ist.

10. Verwendung nach Anspruch 9, **dadurch gekennzeichnet, dass** das Vernetzungshilfsmittel zumindest ein Thiol und/oder ein Selenol, insbesondere jeweils mehrfach funktionelle Derivate davon, sowie Mischungen daraus, ist.

11. Verwendung nach Anspruch 10, **dadurch gekennzeichnet, dass** das Thiol ausgewählt ist aus einer Gruppe umfassend Trimethylolpropan-tris-(3-mercaptopropionat), 1,6-Hexandithiol, sowie Mischungen daraus.

12. Verwendung nach einem der Ansprüche 9 bis 11, **dadurch gekennzeichnet, dass** das Vernetzungshilfsmittel in einem Anteil zugesetzt ist, der ausgewählt ist aus einem Bereich mit einer unteren Grenze von 0,05 Gew.-% und einer oberen Grenze von 20 Gew.-%, bezogen auf den Feststoffgehalt des Latex an der Mischung.

13. Verwendung nach einem der Ansprüche 1 bis 12, **dadurch gekennzeichnet, dass** der Mischung zumindest ein weiteres Vernetzungshilfsmittel zugesetzt ist, ausgewählt aus einer Gruppe umfassend, insbesondere mehrfach funktionelle, Acrylate, wie z.B. Hexandioldiacrylat, Trimethylolpropantriacrylat, Verbindungen mit Vinyl- oder Allylgruppen, wie z.B. Triallyl-Cyanurat, Triallyl-Isocyanurat, sowie Mischungen daraus.

14. Verwendung nach Anspruch 13, **dadurch gekennzeichnet, dass** das weitere Vernetzungshilfsmittel in einem Anteil zugesetzt ist, der ausgewählt ist aus einem Bereich mit einer unteren Grenze von 0,05 Gew.-% und einer oberen Grenze von 20 Gew.-%, bezogen auf den Feststoffgehalt des Latex an der Mischung.

15. Verwendung nach einem der Ansprüche 1 bis 14, **dadurch gekennzeichnet, dass** der Mischung zumindest ein Sensibilisator zugesetzt ist.

16. Verwendung nach Anspruch 15, **dadurch gekennzeichnet, dass** der Sensibilisator ausgewählt ist aus einer Gruppe umfassend organische Farbstoffe wie Eosin, anorganische Pigmente wie Zink-Phthalocyanin oder Titanoxide, sowie Mischungen daraus.

17. Verwendung nach Anspruch 15 oder 16, **dadurch gekennzeichnet, dass** der Sensibilisator in einem Anteil zugesetzt ist, der ausgewählt ist aus einem Bereich mit einer unteren Grenze von 0,1 % und einer oberen Grenze von 50 % des Anteils der Starterkomponente(n).

18. Verwendung nach einem der Ansprüche 1 bis 17, **dadurch gekennzeichnet, dass** zumindest ein Emulgator bzw. Dispergierhilsmittel zugesetzt ist.

19. Verwendung nach Anspruch 18, **dadurch gekennzeichnet, dass** der Emulgator bzw. das Dispergierhilfsmittel ein Tensid ist, insbesondere Polyethylenglykol-sorbitan-monolaurat.

20. Verwendung nach einem der Ansprüche 1 bis 19, **dadurch gekennzeichnet, dass** der Latex ausgewählt ist aus einer Gruppe umfassend Naturkautschuk (NR), Polyisoprene-Latex (IR), Nitrilkautschuk-Latex (NBR), Chloropren-Latex (CR), Styrol-Butadien Latex (SBR), Latices aus Ethylacrylat-Copolymeren (ACM), Latices aus Elastomeren welche durch Re-Emulgieren hergestellt werden, Latices aus funktionellen Copolymeren, Latices hergestellt aus Polymer-Blends, Mischungen daraus.

21. Verwendung nach einem der Ansprüche 1 bis 20, **dadurch gekennzeichnet, dass** der Latex einen Feststoffgehalt aufweist, der ausgewählt ist aus einem Bereich mit einer unteren Grenze von 20 % und einer oberen Grenze von 60 %.

22. Verfahren zur Herstellung eines medizinischen Latexproduktes aus einem vernetzten Elastomer, nach dem eine Mischung umfassend zumindest einen Latex und zumindest eine Starterkomponente zur Auslösung der Vernetzungsreaktion oder ein Latex der zumindest eine Starterkomponente bzw. Startergruppe trägt hergestellt wird, insbesondere in flüssiger Phase, **dadurch gekennzeichnet, dass** eine Mischung entsprechend einem der Ansprüche 1 bis 21 eingesetzt wird und die Latexmischung mit UV-Licht und/oder sichtbaren Licht bestrahlt wird.

23. Verfahren nach Anspruch 22, **dadurch gekennzeichnet, dass** zumindest eine Starterkomponente und/oder zumindest ein Vernetzungshilfsmittel und/oder zumindest ein Sensibilisator vor der Zugabe zu dem zumindest einen Latex zu einer Voremulsion oder Vordispersion voremulgiert oder vordispergiert wird bzw. werden.

24. Verfahren nach Anspruch 23, **dadurch gekennzeichnet, dass** der Voremulsion oder Vordispersion zumindest ein Emulgator bzw. Dispergierhilfsmittel zugesetzt wird.

25. Verfahren nach Anspruch 24, **dadurch gekennzeichnet, dass** die Voremulsion bzw. Vordispersion dem Latex zumindest teilweise vor der Vernetzungsreaktion zugesetzt wird.

26. Verfahren nach Anspruch 24 **dadurch gekennzeichnet, dass** die Voremulsion bzw. Vordispersion dem Latex zumindest teilweise während der Vernetzungsreaktion zudosiert wird.

27. Verfahren nach einem der Ansprüche 22 bis 26, **dadurch gekennzeichnet, dass** die Formgebung des Latexproduktes vor oder nach der Vernetzung durchgeführt wird, und gegebenenfalls nach der Formgebung eine Nachvemetzung durchgeführt wird.

28. Handschuh, insbesondere medizinischer Handschuh oder Operationshandschuh, hergestellt aus zumindest einem, mit einer Starterkomponente vernetzten, durch Elastomermoleküle gebildeten, Elastomer, **dadurch gekennzeichnet, dass** das Elastomer nach einem Verfahren nach einem der Ansprüche 22 bis 27 versetzt ist und die Starterkomponente bzw. die bei der Fotolyse entstehenden Zersetzungsprodukte aus der Starterkomponente an den Elastomermolekülen kovalent gebunden ist bzw. sind.

29. Handschuh nach Anspruch 28, **dadurch gekennzeichnet, dass** an den Elastomermolekülen zumindest ein Vernetzungshilfsmittel, insbesondere ein multifunktionelles Thiol, immobilisiert, insbesondere kovalent gebunden, ist.

30. Handschuh nach einem der Ansprüche 28 oder 29, **dadurch gekennzeichnet, dass** das Elastomer ausgewählt ist aus einer Gruppe umfassend Naturkautschuk (NR), Polyisoprene-Latex (IR), Nitrilkautschuk-Latex (NBR), Chloropren-Latex (CR), Styrol-Butadien Latex (SBR), Latices aus Ethylacrylat-Copolymeren (ACM), Latices aus Elastomeren welche durch Re-Emulgieren hergestellt werden, Latices aus funktionellen Copolymeren, Latices hergestellt aus Polymer-Blends, Mischungen daraus.

## Claims

1. Use of a mixture comprising at least one latex and at least one photoinitiator, which forms a reactive species when illuminated with electromagnetic radiation in the ultraviolet (UV light) and/or visible spectral range in order to initiate the cross-linking reaction, and the at least one photoinitiator or at least one photoinitiator group is optionally part of the latex molecule, and optionally other additives for producing a glove, surgical glove, condom or a medical latex product.

2. Use as claimed in claim 1, **characterised in that** the starter component is selected from a group comprising compounds which split into radicals under the effect of UV radiation, in particular benzoin derivatives, methylol benzoin derivatives, 4-benzoyl-1,3-dioxolan derivatives, benzil ketals, dialkoxyacetophenones, hydroxyalkyl phenones, aminoalkyl phenones, acyl phosphinoxides, o-acyl-α-oximino ketones, peroxides, halogenated acetophenone derivatives, phenylglyoxylates, and mixtures thereof.

3. Use as claimed in one of claims 1 or 2, **characterised in that** the starter component is selected from a group comprising compounds which split off hydrogen and form radicals under the effect of UV radiation, in particular benzophenone derivatives, Michler=s ketones, xanthone derivatives, thioxanthone derivatives, anthraquinone derivatives, benzil, diinone compounds and mixtures thereof.

4. Use as claimed in one of claims 1 to 3, **characterised in that** the starter component is selected from a group comprising oligomeric photoinitiators, photoinitiators with vinyl and/or acryl groups, macro-molecules, in particular polymerised or co-polymerised photoinitiators, such as oligo [2-hydroxy-2-methyl-1-[4-(1-methylvinyl)phenyl] propanone], polymerisable photoinitiators, polymer-bonded photoinitiators, photoinitiators which bond to a polymer or rubber matrix when irradiated, as well as polymerised vinyl, allyl, acryl or methacryl derivatives of: benzoin derivatives, methylol benzoin derivatives, 4-benzoyl-1,3-dioxolane derivatives, benzil ketals, dialkoxyacetophenones, hydroxyalkyl phenones, aminoalkyl phenones, acyl phosphinoxides, o-acyl-α-oximino ketones, peroxides, halogenated acetophenone derivatives, phenylglyoxylates, benzo-phenone derivatives, Michler=s ketones, xanthone derivatives, thioxanthone derivatives, anthraquinone derivatives, benzil, diinone compounds and mixtures thereof.

5. Use as claimed in one of claims 1 to 4, **characterised in that** the starter component is selected from a group comprising photoinitiators which can be excited with visible light, in particular titanocenes, 1,2-diketones, such as benzil, derivatives of acyl phosphinoxides, substituted derivatives of stilbene, distyryl benzol and mixtures thereof.

6. Use as claimed in one of claims 1 to 5, **characterised in that** at least one hydrogen donor such as an amine or thiol is added to the starter component.

7. Use as claimed in one of claims 1 to 6, **characterised in that** the starter component is a compound selected from a group comprising 2,4,6-trimethylbenzoylphenyl phosphinic acid ethyl ester, 2-hydroxy-2-methyl-1-phenyl propanone.

8. Use as claimed in one of claims 1 to 7, **characterised in that** the at least one starter component or a mixture of several starter components is added in a quantity selected from a range with a lower limit of 0.05 % by weight and an upper limit of 20 % by weight relative to the solid content of the latex in the mixture.

9. Use as claimed in one of claims 1 to 8, **characterised in that** at least one cross-linking agent is added to the mixture in addition.

10. Use as claimed in claim 9, **characterised in that** the cross-linking agent is at least one thiol and/or a selenol, in particular multiple functional derivatives thereof respectively, and mixtures thereof.

11. Use as claimed in claim 10, **characterised in that** the thiol is selected from a group comprising trimethylol propene-tris-(3-mercaptoproprianate), 1,6-hexane dithiol, and mixtures thereof.

12. Use as claimed in one of claims 9 to 11, **characterised in that** the cross-linking agent is added in a quantity selected from a range with a lower limit of 0.05 % by weight and an upper limit of 20 % by weight relative to the solid content of the latex in the mixture.

13. Use as claimed in one of claims 1 to 12, **characterised in that** at least one other cross-linking agent is added to the mixture, selected from a group comprising in particular multiple functional acrylates, such as hexane dioldiacrylate, trimethylol propane triacrylate, compounds with vinyl or allyl groups, such as triallyl cyanurate, triallyl isocyanurate, and mixtures thereof.

14. Use as claimed in claim 12, **characterised in that** the other cross-linking agent is added in a quantity selected from a range with a lower limit of 0.05 % by weight and an upper limit of 20 % by weight relative to the solid content of the latex in the mixture.

15. Use as claimed in one of claims 1 to 14, **characterised in that** at least one sensitizer is added to the mixture.

16. Use as claimed in claim 15, **characterised in that** the sensitizer is selected from a group comprising organic dyes such as eosin, inorganic pigments such as zinc phthalocyanine or titanium oxides, and mixtures thereof.

17. Use as claimed in claim 15 or 16, **characterised in that** the sensitizer is added in a quantity selected from a range with a lower limit of 0.1 % by weight and an upper limit of 50 % by weight of the quantity of starter component(s).

18. Use as claimed in one of claims 1 to 17, **characterised in that** at least one emulsifier or dispersing agent is added.

19. Use as claimed in claim 18, **characterised in that** the emulsifier or dispersing agent is a surfactant, in particular polyethylene glycol-sorbitan monolaurate.

20. Use as claimed in one of claims 1 to 19, **characterised in that** the latex is selected from a group comprising natural rubber (NR), polyisoprene latex (IR), nitrile rubber latex (NBR), chloroprene latex (CR), styrene-butadiene latex (SBR), latexes of ethyl acrylate copolymers (ACM), latexes of elastomers produced by re-emulsification, latexes of functional copolymers, latexes produced from polymer blends, and mixtures thereof.

21. Use as claimed in one of claims 1 to 20, **characterised in that** the latex has a solid content selected from a range with a lower limit of 20 % and an upper limit of 60 %.

22. Method of producing a medical latex product from a cross-linked elastomer, whereby a mixture comprising at least one latex and at least one starter component for initiating the cross-linking reaction or a latex of the at least one starter component or starter group is produced, in particular in liquid phase, **characterised in that** a mixture as claimed in one of claims 1 to 21 is used and the latex mixture is irradiated with UV light and/or visible light.

23. Method as claimed in claim 22, **characterised in that** at least one starter component and/or at least one cross-linking agent and/or at least one sensitizer is or are pre-emulsified or pre-dispersed to obtain a pre-emulsion or pre-dispersion prior to being added to the at least one latex.

24. Method as claimed in claim 23, **characterised in that** at least one emulsifier or dispersing agent is added to the pre-emulsion or pre-dispersion.

25. Method as claimed in claim 24, **characterised in that** the pre-emulsion or pre-dispersion is at least partially added to the latex prior to the cross-linking reaction.

26. Method as claimed in claim 24, **characterised in that** the pre-emulsion or pre-dispersion is at least partially metered into the latex during the cross-linking reaction.

27. Method as claimed in one of claims 22 to 26, **characterised in that** the latex product is formed before or after cross-linking and a final cross-linking is optionally run after forming.

28. Glove, in particular a medical glove or surgical glove, produced from at least one elastomer in the form of elastomer molecules cross-linked with a starter component, **characterised in that** the elastomer is cross-linked by a method as claimed in one of claims 22 to 27 and the starter component or the decomposition products created from the starter component during photolysis is or are covalently bonded to the elastomer molecules.

29. Glove as claimed in claim 28, **characterised in that** at least one cross-linking agent, in particular a multi-functional thiol, is immobilised on the elastomer molecules, in particular covalently bonded.

30. Glove as claimed in one of claims 28 or 29, **characterised in that** the elastomer is selected from a group comprising natural rubber (NR), polyisoprene latex (IR), nitrile rubber latex (NBR), chloroprene latex (CR), styrene-butadiene latex (SBR), latexes of ethyl acrylate copolymers (ACM), latexes of elastomers produced by re-emulsification, latexes of functional copolymers, latexes produced from polymer blends, and mixtures thereof.

## Revendications

1. Utilisation d'un mélange comprenant au moins un latex et au moins un photoinitiateur qui forme une espèce réactive par exposition à un rayonnement électromagnétique dans la plage ultraviolette (lumière UV) et/ou visible du spectre, pour le déclenchement de la réaction de réticulation, sachant que ledit au moins un photoinitiateur, respectivement au moins un groupe de photoinitiateurs est le cas échéant une partie des molécules de latex, ainsi que le cas échéant d'autres substances auxiliaires, à la fabrication d'un gant, d'un gant pour interventions chirurgicales, d'un préservatif ou d'un produit médical en latex.

2. Utilisation selon la revendication 1, **caractérisée en ce que** le composant initiateur est choisi parmi le groupe comportant des composés qui, sous l'influence d'un rayonnement UV, se scindent en radicaux, en particulier des dérivés de la benzoïne, des dérivés de la méthylolbenzoïne, des dérivés du 4-benzoyl-1,3-dioxolane, des benzylcétals, des dialcoxyacétophénones, des hydroxyalkylphénones, des aminoalkylphénones, des oxydes d'acylphosphine, des o-acyl-α-oximinocétones, des peroxydes, des dérivés halogénés d' acétophénone, des phénylglyoxylates, ainsi que des mélanges de ceux-ci.

3. Utilisation selon l'une quelconque des revendications 1 ou 2, **caractérisée en ce que** le composant initiateur est choisi parmi le groupe comprenant des composés qui, sous l'influence du rayonnement UV, éliminent de l'hydrogène moyennant formation de radicaux, en particulier des dérivés de la benzophénone, des cétones de Michler, des dérivés de la xanthone, des dérivés de la thioxanthone, des dérivés de l'anthraquinone, le benzile, les composés de la diinone, ainsi que des mélanges de ceux-ci.

4. Utilisation selon l'une quelconque des revendications 1 à 3, **caractérisée en ce que** le composant initiateur est choisi parmi le groupe comprenant des photoinitiateurs oligomères, des photoinitiateurs avec des groupes vinyle et/ou acryle, des macromolécules, en particulier des photoinitiateurs polymérisés ou copolymérisés comme par exemple les oligo [2-hydroxy-3-méthyl-1-[4-(1-méthylvinyl)phényl] propanones], des photoinitiateurs polymérisables, des photoinitiateurs liés à des polymères, des photoinitiateurs qui se lient moyennant rayonnement à une matrice de polymère, respectivement de caoutchouc, ainsi que des dérivés polymérisés de vinyle, d'allyle, d'acryle ou de méthacryle de: dérivés de la benzoïne, dérivés de la méthylolbenzoïne, dérivés du 4-benzoyl-1,3-dioxolane, des benzylcétals, des dialcoxyacétophénones, des hydroxyalkylphénones, des aminoalkylphénones, des oxydes d'acylphosphine, des o-acyl-α-oximinocétones, des peroxydes, des dérivés halogénés d'acétophénone, des phénylglyoxylates, des dérivés de la benzophénone, des cétones de Michler, des dérivés de la xanthone, des dérivés de la thioxanthone, des dérivés de l' anthraquinone, le benzile, les composés de la diinone, ainsi que des mélanges de ceux-ci.

5. Utilisation selon l'une quelconque des revendications 1 à 4, **caractérisée en ce que** le composant initiateur est choisi parmi un groupe comprenant des photoinitiateurs qui peuvent être excités avec de la lumière visible, en particulier des titanocènes, des 1,2-dicétones comme par exemple le benzile, des dérivés d'oxydes d'acylphosphine, des dérivés substitués du stilbène, du distyrylbenzène, ainsi que des mélanges de ceux-ci.

6. Utilisation selon l'une quelconque des revendications 1 à 5, **caractérisée en ce qu'**au composant initiateur est ajouté au moins un donneur d'hydrogène comme par exemple une amine ou un thiol.

7. Utilisation selon l'une quelconque des revendications 1 à 6, **caractérisée en ce que** le composant initiateur est un composé choisi parmi un groupe comprenant l'ester éthylique de l'acide 2,4,6,-triméthylbenzoylphénylhypophosphoreux, la 2-hydroxy-2-méthyl-1-phénylpropanone.

8. Utilisation selon l'une quelconque des revendications 1 à 7, **caractérisée en ce que** ledit au moins un composant initiateur ou un mélange de plusieurs composants initiateurs est ajouté en une quote-part qui est choisie dans un intervalle avec une limite inférieure de 0,05 % en poids et une limite supérieure de 20 % en poids, rapportées à la teneur en solide du latex dans le mélange.

9. Utilisation selon l'une quelconque des revendications 1 à 8, **caractérisée en ce qu'**au moins un auxiliaire de réticulation est ajouté en plus au mélange.

10. Utilisation selon la revendication 9, **caractérisée en ce que** l'auxiliaire de réticulation est au moins un thiol et/ou un sélénol, en particulier des dérivés plurifonctionnels respectifs de ceux-ci, ainsi que des mélanges de ceux-ci.

11. Utilisation selon la revendication 10, **caractérisée en ce que** le thiol est choisi parmi un groupe comprenant le triméthylolpropane-tris-(3-mercaptopropionate), le 1,6-hexane dithiol, ainsi que des mélanges de ceux-ci.

12. Utilisation selon l'une quelconque des revendications 9 à 11, **caractérisée en ce que** l'auxiliaire de réticulation est ajouté en une quote-part qui est choisie dans un intervalle avec une limite inférieure de 0,05 % en poids et une limite supérieure de 20 % en poids, rapportées à la teneur en solide du latex dans le mélange.

13. Utilisation selon l'une quelconque des revendications 1 à 12, **caractérisée en ce qu'**est ajouté au mélange au moins un autre auxiliaire de réticulation, choisi parmi un groupe comprenant des acrylates, comme par exemple le diacrylate d'hexane diol, le triacrylate de triméthylolpropane, en particulier plurifonctionnels, des composés avec des groupes vinyles ou allyles comme par exemple le cyanurate de triallyle, l'isocyanurate de triallyle, ainsi que des mélanges de ceux-ci.

14. Utilisation selon la revendication 13, **caractérisée en ce que** l'autre auxiliaire de réticulation est ajouté en une quantité qui est choisie dans un intervalle avec une limite inférieure de 0,05 % en poids et une limite supérieure de 20 % en poids, rapportées à la teneur en solide du latex dans le mélange.

15. Utilisation selon l'une quelconque des revendications 1 à 14, **caractérisée en ce qu'**au moins un sensibilisateur est ajouté au mélange.

16. Utilisation selon la revendication 15, **caractérisée en ce que** le sensibilisateur est choisi parmi un groupe comprenant des colorants organiques comme l'éosine, des pigments inorganiques comme la phtalocyanine de zinc ou des oxydes de titane, ainsi que des mélanges de ceux-ci.

17. Utilisation selon la revendication 15 ou 16, **caractérisée en ce que** le sensibilisateur est ajouté en une proportion qui est choisie dans un intervalle avec une limite inférieure de 0,1 % et une limite supérieure de 50 % de la quote-part du ou des composants initateurs.

18. Utilisation selon l'une quelconque des revendications 1 à 17, **caractérisée en ce qu'**au moins un émulsionnant, respectivement un dispersant, est ajouté.

19. Utilisation selon la revendication 18, **caractérisée en ce que** l'émulsionnant, respectivement le dispersant est un tensioactif, en particulier un monolaurate de polyéthylène glycol sorbitane.

20. Utilisation selon l'une quelconque des revendications 1 à 19, **caractérisée en ce que** le latex est choisi parmi un groupe comprenant le caoutchouc naturel (NR), le latex de poly-isoprène (IR), le latex de caoutchouc nitrile (NBR), le latex de chloroprène (CR), le latex de styrène-butadiène (SBR), les latex de copolymère d'acrylate d'éthyle (ACM), les latex d'élastomères qui sont fabriqués par remise en émulsion, les latex de copolymères fonctionnels, les latex de mélanges de polymères, et des mélanges de ceux-ci.

21. Utilisation selon l'une quelconque des revendications 1 à 20, **caractérisée en ce que** le latex présente une teneur en solide qui est choisie dans un intervalle avec une limite inférieure de 20 % et une limite supérieure de 60 %.

22. Procédé de fabrication d'un produit médical en latex en un élastomère réticulé, selon lequel un mélange comprenant au moins un latex et au moins composant initiateur pour déclencher la réaction de réticulation ou un latex qui porte au moins un composant initiateur, respectivement un groupe de départ, est préparé, en particulier en phase liquide, **caractérisé en ce qu'**un mélange correspondant à une quelconque des revendications 1 à 21 est utilisée et **en ce que** le mélange de latex est exposé à de la lumière UV et/ou à de la lumière visible.

23. Procédé selon la revendication 22, **caractérisé en ce qu'**avant l'addition audit au moins un latex, au moins un composant initiateur et/ou au moins un auxiliaire de réticulation et/ou au moins un sensibilisateur sont pré-émulsionnés ou prédispersés pour donner une pré-émulsion ou une prédispersion.

24. Procédé selon la revendication 23, **caractérisé en ce qu'**au moins un émulsionnant, respectivement un auxiliaire de dispersion, est ajouté à la pré-émulsion ou prédispersion.

25. Procédé selon la revendication 24, **caractérisé en ce que** la pré-émulsion, respectivement la prédispersion est ajoutée au latex au moins en partie avant la réaction de réticulation.

26. Procédé selon la revendication 24, **caractérisé en ce que** la pré-émulsion, respectivement la prédispersion, est dosée dans le latex au moins en partie pendant la réaction de réticulation.

27. Procédé selon l'une quelconque des revendications 22 à 26, **caractérisé en ce que** la mise en forme du produit en latex est effectuée avant ou après la réticulation et **en ce qu'**une post-réticulation est effectuée le cas échéant après la mise en forme.

28. Gant, en particulier gant médical ou gant pour interventions chirurgicales, fabriqué en au moins un élastomère réticulé avec un composant initiateur, formé par des molécules d'élastomère, **caractérisé en ce que** l'élastomère est réticulé moyennant un procédé selon l'une quelconque des revendications 22 à 27 et **en ce que** le composant initiateur, respectivement les produits de décomposition apparaissant lors de la photolyse, sont liés par covalence aux molécules d'élastomère.

29. Gant selon la revendication 28, **caractérisé en ce qu'**au moins un auxiliaire de réticulation, en particulier un thiol multifonctionnel, est immobilisé, en particulier lié par covalence, sur les molécules d'élastomère.

30. Gant selon l'une quelconque des revendications 28 ou 29, **caractérisé en ce que** l'élastomère est choisi parmi un groupe comprenant le caoutchouc naturel (NR), le latex de poly-isoprène (IR), le latex de caoutchouc nitrile (NBR), le latex de chloroprène (CR), le latex de styrène-butadiène (SBR), les latex de copolymère d'acrylate d'éthyle (ACM), les latex d'élastomères qui sont fabriqués par remise en émulsion, les latex de copolymères fonctionnels, les latex de mélanges de polymères, et des mélanges de ceux-ci.
